(12) **EUROPÄISCHE PATENTSCHRIFT**

(45) Veröffentlichungstag und Bekanntmachung des Hinweises auf die Patenterteilung:
**04.10.2017  Patentblatt 2017/40**

(21) Anmeldenummer: **10719908.5**

(22) Anmeldetag: **21.04.2010**

(51) Int Cl.:
*A61M 1/16* *(2006.01)*

(86) Internationale Anmeldenummer:
**PCT/EP2010/002454**

(87) Internationale Veröffentlichungsnummer:
**WO 2010/121805 (28.10.2010 Gazette 2010/43)**

(54) **DIALYSEMASCHINE, VERFAHREN UND VORRICHTUNG FÜR EINE DIALYSEMASCHINE**

DIALYSIS MACHINE, METHOD AND DEVICE FOR A DIALYSIS MACHINE

DIALYSEUR, PROCÉDÉ ET DISPOSITIF POUR DIALYSEUR

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **23.04.2009  DE 102009018649**

(43) Veröffentlichungstag der Anmeldung:
**29.02.2012  Patentblatt 2012/09**

(73) Patentinhaber: **Fresenius Medical Care Deutschland GmbH**
**61352 Bad Homburg (DE)**

(72) Erfinder:
 • **GROSS, Malte**
 **97464 Niederwerrn (DE)**

 • **MAIERHOFER, Andreas**
 **97422 Schweinfurt (DE)**

(74) Vertreter: **Herrmann, Uwe et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**US-A- 5 091 094     US-A1- 2001 004 523**

 • **SARGENT J A ET AL: "PRINCIPLES AND BIOPHYSICS OF DIALYSIS" 1. Januar 1989 (1989-01-01), REPLACEMENT OF RENAL FUNCTION BY DIALYSIS, KLUWER ACADEMIC PUBL, XX, PAGE(S) 87 - 143 , XP000564853 das ganze Dokument**

**Beschreibung**

Die vorliegende Erfindung betrifft eine Dialysemaschine und ein Verfahren zum Betrieb einer Dialysemaschine. Bei der Hämodialyse findet ein Stoffaustausch zwischen Patienten und Dialysat statt. Dessen Ausmaß wird bestimmt durch die stoffspezifische Dialyseeffizienz, die durch Dialysatortyp, Behandlungsart (HD, HDF, HF, usw.), Blut-, Dialysat- und Substituatfluss gegeben ist, sowie durch den um den Donnanfaktor korrigierten Konzentrationsgradienten zwischen Blutplasma und Dialysat. Hierbei wird die Stoffkonzentration im Blutplasma durch den Stoffaustausch zwischen Blut und anderen Kompartimenten (intrazellulär, interstitiell) beeinflusst. Je größer dabei das stoffspezifische Verteilungsvolumen ist, um so geringer ist die Konzentrationsänderung im Blutplasma bei gleichem Stoffaustausch über den Dialysator.

Bei einer unendlich langen Dialyse würde bei Abwesenheit eines Metabolismus des betrachteten Stoffes im Patienten die Konzentration im Blutplasma die Konzentration des Stoffes im Dialysat bzw. bei Ionen die durch das Donnangleichgewicht gegebene Konzentration erreichen, wobei die stoffspezifische Kinetik durch die Behandlungsparameter und physiologischen Parameter des Patienten bestimmt wird. Weicht die Stoffkonzentration im Dialysat stark von derjenigen im Blutplasma ab, was insbesondere dann der Fall ist, wenn der betreffende Stoff zwar im Blut aber nicht im Dialysat vorhanden ist (z. B. Dialysat ohne Glucose, Kalium, Calcium), so können während der Dialyse im Patientenplasma Stoffkonzentrationen erreicht werden, die zu kritischen Symptomen im Patienten führen können. Ebenso kann es sein, dass die erreichte Stoffkonzentration im Patientenplasma zwar an sich vom Patienten toleriert wird, jedoch die Geschwindigkeit des Stoffentzuges zu hoch ist, was zu Symptomen führen kann (z. B. das Disäquilibrium- Symptom bei zu schnellem Entzug von Harnstoff). Die Art und Schwere der Symptome kann vom Ausmaß der Konzentrationsverschiebung einzelner Stoffe oder einer durch mehrere Stoffe bedingten Summengröße (z. B. Osmolalität) abhängen.

Zur Vermeidung von intradialytischen Symptomen ist es daher wünschenswert, das Erreichen von kritischen Konzentrationen einzelner Stoffe oder von Summengrößen mehrerer Substanzen im Patienten rechtzeitig zu detektieren bzw. vorherzusagen und damit kritische Situationen zu vermeiden.

Aus der US 2002/0099282 A1 ist für die Diabetestherapie ein Verfahren bekannt, bei dem der Glukosekonzentrationsverlauf im Patienten nach Eingabe eines initialen Wertes abgeschätzt wird.

Die US 2003/0208113 A1 beschreibt ein System für die Diabetestherapie zur Abschätzung des Glukosekonzentrationsverlaufs im Patienten unter Verwendung von Messwerten sowie weiterer vom Patienten eingegebener Informationen wie Nahrungsaufnahme. Beim Auftreten von kritischen Glukosekonzentrationen werden an den Patienten Handlungsanweisungen ausgegeben, um seine Glukosekonzentration in einen unkritischen Bereich einzustellen.

Beide Systeme haben jedoch keinen Bezug zur Dialysebehandlung.

Aus US 5,091,094 ist ein Dialysesystem bekannt, bei dem mittels eines Modells zum Stoffaustausch über die semipermeable Membran die Diffusionsrate von gelösten Stoffen ermittelt werden kann. Dazu wird zunächst die Konzentration von Serum Albumin im Blut bestimmt. Weiterhin wird die Konzentration verschiedener im Blut gelöster Stoffe abgeschätzt. Die EP 0 942 759 B1 betrifft ein System zur Vermeidung von intradialytischen Symptome, wobei das Disäquilibrium-Symptom bei zu schnellem Entzug von Harnstoff verhindert werden soll. Dabei wird ein Harnstoffmonitor im abfließenden Dialysat verwendet, um die Dialyseeffizienz zu bestimmen. Weiter wird eine empirisch bestimmte Kurve für den Patienten herangezogen, die die maximal für den Patienten verträgliche Dialyseeffizienz beschreibt, um die Dialyseeffizienz derart einzuregeln, dass die Werte der Kurve nicht erreicht werden. Die EP 0 942 759 erlaubt es jedoch nicht, bereits im Vorfeld abzuschätzen, ob es im Laufe der Behandlung zur einer kritischen Stoffkonzentration eines gelösten Stoffes im Patienten kommen wird. Durch die Fokussierung auf den Harnstoffentzug ist es darüber hinaus nicht möglich, weitere Stoffe zu berücksichtigen, die ggf. zu intradialytischen Symptomen führen können. Beispielsweise ist von Nachteil, dass zwar die Ist-Werte für den Harnstoff ihre kritische Kurve nicht erreichen, jedoch andere Stoffe wie Natriumionen, Calciumionen oder Glukose in einem kritischen Bereich sein können, so dass trotzdem intradialytische Symptome auftreten.

Es ist daher die Aufgabe der vorliegenden Erfindung, eine Dialysemaschine der eingangs genannten Art in vorteilhafter Weise weiterzubilden, insbesondere dahingehend, dass erkannt werden kann, ob es im Laufe der Behandlung wahrscheinlich zu einer kritischen Stoffkonzentration im Patienten kommen wird und die Betriebssicherheit der Dialysemaschine erhöht wird.

Diese Aufgabe wird erfindungsgemäß gelöst durch eine Dialysemaschine mit den Merkmalen des Anspruchs 1. Danach ist vorgesehen, dass eine Dialysemaschine wenigstens ein Eingabemittel zur Eingabe von Parametern für die Dialysebehandlung aufweist. Weiter weist die Dialysemaschine eine Berechnungseinheit auf, mittels derer unter Vorhaltung eines Modells zumindest unter Einbeziehung der zuvor eingegebenen Parameter für wenigstens eine Konzentration eines gelösten Stoffes der durch die Dialysebehandlung zu erwartende Verlauf der Konzentrationsänderung und/oder der zu erwartende Endwert der Konzentration dieses Stoffes berechenbar und auswertbar ist. Ferner ist eine Steuer- und/oder Regelungseinheit vorgesehen, mittels derer mittelbar und/oder unmittelbar in Abhängigkeit von der Auswertung des zu erwartenden Verlaufs der Konzentrationsänderung und/oder des zu erwartenden Endwertes der Konzentration des Stoffes über Ausgabemittel wenigstens ein Signal und/oder eine Anzeige ausgebbar ist und/oder die Dialysebehandlung bzw. die maschinenseitigen Einstellungen für die Dialysebehandlung mittelbar und/oder unmittelbar beeinflussbar ist/sind.

Die Auswertung umfasst vorzugsweise eine Prognose des zu erwartenden Verlaufs der Konzentrationsänderung und/oder des zu erwartenden Endwertes des ausgewerteten Stoffes während der Dialysebehandlung.

Dadurch kann in besonders vorteilhafter Weise die Betriebssicherheit einer Dialysemaschine erhöht werden, da beispielsweise vor Beginn, aber auch während der Dialysebehandlung, anhand von maschinenseitigen Berechnungen und Auswertungen erkannt werden kann, ob bei einer Dialysebehandlung mit dem aktuell vorgegebenen Setup bzw. den aktuell vorgegebenen Behandlungsparametern und -umständen ein ggf. nachteiliger Verlauf der Konzentrationsänderung eines gelöstes Stoffes und/oder ein zu hoher oder zu niedriger Wert eines gelösten Stoffes oder einer durch mehrere Stoffe bedingten Summengröße in Folge der Dialysebehandlung im vaskulären Blutkreislauf des Patienten zu erwarten ist.

Die Eingabemittel können dabei insbesondere eine Datenübertragungsschnittstelle, ein Dateneingabemittel wie Tastatur oder Touchscreen oder ähnliche Mittel umfassen oder als solche ausgeführt sein. Es kann vorgesehen sein, dass das Eingabemittel zugleich auch Ausgabemittel ist, etwa ein Benutzerinterface einer Dialysemaschine.

Grundsätzlich ist denkbar, dass Berechnungseinheit und Steuer- und/oder Regelungseinheit Bestandteile einer zentralen Maschinensteuerung sind und ggf. aus Sicherheitsgründen redundant vorhanden sind. Insbesondere kann vorteilhafterweise vorgesehen sein, die zentrale Maschinensteuerung mit Berechnungseinheit und Steuer- und/oder Regelungseinheit redundant auszuführen.

Darüber hinaus ist denkbar, dass eine Speichereinheit vorgesehen ist, in der wenigstens ein Grenzwert für einen Parameter einspeicherbar ist, bei dessen Über- oder Unterschreitung ein kritischer Wert vorliegt, insbesondere Grenzwerte für zu erwartenden Verlauf und/oder zu erwartenden Endwert der Konzentration des wenigstens einen gelösten Stoffes, wobei zwischen Speichereinheit und Berechnungseinheit eine Datenaustauschverbindung besteht und wobei mittels der Berechnungseinheit erkennbar ist, dass bei Unter- oder Überschreitung des Grenzwerts ein kritischer Wert vorliegt und/oder zu erwarten ist. Dadurch ergibt sich der Vorteil, dass das Setzen von Grenzwerten einfach möglich ist und einmal gesetzte Grenzwerte für weitere Behandlungen wiederverwendet werden können. Des Weiteren ist von Vorteil, dass durch die Einspeicherung von Grenzwerten kritische Werte und damit problematische Behandlungssituationen automatisiert durch die Maschine durch eine automatisiert erstellte Prognose des Behandlungsverlaufs und des Ergebnisses der Behandlung vor der Behandlung aber auch während der Behandlung erkannt werden können. Durch die Speichereinheit ergibt sich grundsätzlich weiter der Vorteil, dass Parameter, die mittelfristig konstant sind - etwa das stoffspezifische Verteilungsvolumen eines Patienten, Medikation, Risikofaktoren, Unverträglichkeiten, Antikoagulation und/oder verschriebene Dialysatzusammensetzung - eingespeichert vorgehalten werden können und nicht für jede Behandlung erneut eingegeben werden müssen.

Es ist möglich, dass mittels des Eingabemittels initiale Blutwerte, Verteilungsvolumen des Patienten und/oder Grenzwerte für einen Parameter eingebbar sind. Dadurch ergibt sich der Vorteil, bereits gesetzte Grenzwerte einfach ändern zu können, etwa um eine Anpassung der Behandlung durchzuführen.

Es kann vorgesehen sein, dass die Speichereinheit ein entnehmbares Speichermedium umfasst und/oder mit einem entnehmbaren Speichermedium verbindbar ist, wobei auf das entnehmbare Speichermedium wenigstens ein Parameter für die Dialysebehandlung einspeicherbar ist und/oder dass mittels des entnehmbaren Speichermediums, das als Teil der Eingabemittel ausgeführt ist, wenigstens ein Parameter für die Dialysebehandlung in die Dialysemaschine eingebbar ist. Dadurch ist es einfach möglich, Parameter für die Behandlung einzugeben. Insbesondere wird es dadurch erleichtert, den Patienten an mehreren Maschinen behandeln zu können. Auch eine Behandlung in mehreren Dialysezentren, wie dies beispielsweise bei Urlauben des Patienten der Fall sein kann, ist hierdurch ohne weiteres möglich. Das entnehmbare Speichermedium kann vorteilhafterweise eine entnehmbare Patientenspeicherkarte sein. Denkbar ist, dass es sich dabei um die Gesundheits- und/oder Versichertenkarte des Patienten handelt. Genausogut kann es sich um eine spezielle Karte handeln, die z. B. durch den Maschinenhersteller bereitgestellt und durch z. B. das Dialysezentrum oder die Behandlungsstation an den Patienten ausgegeben und gepflegt wird. Bei der erfindungsgemäßen Dialysemaschine ist/sind mittels der Steuerungs- und/oder Regelungseinheit die Dialysebehandlung, insbesondere die maschinenseitigen Einstellungen für die Dialysebehandlung, hinsichtlich Behandlungsdauer, Dialysatzusammensetzung, Einstellung der Flussraten im Blutkreislauf und/oder Dialysatkreislauf und/oder Einstellung der Art der Dialysebehandlung beeinflussbar, wobei vorzugsweise die Dialysatzusammensetzung durch Ansteuerung einer Dosiereinheit für Konzentrat von Dialysatbestandteilen und/oder die Flussraten durch Ansteuerung von Pumpen des Blutkreislaufs und/oder Dialysatkreislaufs beeinflussbar ist/sind. Hierzu ist vorteilhaft denkbar, dass entsprechende Komponenten der Dialysemaschine mit der Steuerungs- und/oder Regelungseinheit und/oder der Berechnungseinheit in Signalverbindung stehen, die die aktuellen Betriebsparameter mittelbar und/oder unmittelbar erfasst und/oder auswerten kann und über entsprechende Ansteuersignale ggf. die jeweiligen Komponenten gemäß der gewünschten Beeinflussung der Dialysebehandlung ansteuert.

Außerdem ist es möglich, dass mittels der Ausgabemittel Informationen zu einsetzbaren Komponenten für die Dialysemaschine, wählbaren Parameterbereichen für die Dialysebehandlung und/oder Warnsignale ausgebbar sind. Einsetzbare Komponenten können beispielsweise Typ(en) des Dialysators oder des Dialysatkonzentrats sein. Denkbar ist, dass bei Ermittlung des Erreichens eines kritischen Wertes alternative Komponenten maschinenseitig vorgeschlagen werden können, bei deren Einsatz ein kritischer Wert nicht erreicht wird. Die wählbaren Parameterbereiche können beispielsweise die Einstellung der Dialysatzusammensetzung und/oder die Flussraten im Dialysat- und/oder Blutkreislauf umfassen.

Ferner kann vorgesehen sein, dass mittels der Regelungs- und/oder Steuerungsmittel die Dialysebehandlung abschaltbar und/oder ein Starten der Dialysebehandlung verhinderbar ist.

Darüber hinaus ist denkbar, dass der wenigstens eine kritische Wert ein Wert ist, bei dem Hypoglycämie, Hypocalciämie, Hypokaliämie, Alkalose bedingt durch Bikarbonat, ein Disäquilibrium, insbesondere ein Disäquilibrium hinsichtlich Harnstoff und/oder Blutalkohol, und/oder Hyponatriämie beim zu behandelnden Patienten eintreten würde. Dabei kann es sich um bekannte Werte aus der Literatur oder um anhand der Patientenhistorie empirisch ermittelte Werte handeln.

Weiter ist möglich, dass bei Erkennung eines kritischen Wertes eine Warnmeldung ausgebbar ist und/oder eine Vorschlagsmeldung betreffend einen Konzentratwechsel für das Dialysat ausgebbar ist und/oder dass bei Erkennung eines kritischen Wertes der Start der Dialysebehandlung blockiert ist. Von Vorteil ist dabei, dass durch eine Blockade des Starts der Dialysebehandlung das Behandlungspersonal die eingestellten und maschinenseitig als nicht korrekt oder verbesserungsbedürftig erkannten Betriebsparameter erneut prüfen und abändern muss. Die Behandlungssicherheit kann hierdurch erhöht werden.

Ferner kann vorgesehen sein, dass bei Erkennung eines kritischen Wertes während der Dialysebehandlung eine Warnmeldung betreffend die gesonderte Bestimmung aktueller Blutparameter des Patienten ausgebbar ist, insbesondere hinsichtlich der Bestimmung von Blutzucker, Calcium, Kalium, Natrium, Harnstoff, Alkohol und/oder pH-Wert. Eine derartige Warnmeldung kann beispielsweise darin bestehen, dass das Behandlungspersonal aufgefordert wird, aktuelle Blutparameter des Patienten gesondert zu bestimmen. Eine etwaige Anpassung der Behandlung kann dann in diesem Fall durch das Behandlungspersonal vorgenommen werden.

Außerdem ist denkbar, dass die Dialysemaschine bei Erkennung eines kritischen Wertes während der Dialysebehandlung mittels der Steuerungs- und/oder Regelungsmittel von einem Dialyseverfahren auf ein Filtrationsverfahren, vorzugsweise die isolierte Ultrafiltration (Iso-Ultrafiltration), umschaltbar ist. Diese Funktion ist insbesondere dann von Vorteil, wenn der Verlauf der Konzentrationsänderung eines z. B. dialysepflichtigen gelösten Stoffes eine erhöhte Wahrscheinlichkeit für das Auftreten eines Disäquilibriums erwarten lässt. Durch das Umschalten auf Iso-Ultrafiltration wird der Verlauf der Konzentrationsänderung dieses Stoffes angepasst, so dass die Wahrscheinlichkeit des Auftretens eines Disäquilibriums vermindert werden kann.

Weiter kann vorgesehen sein, dass wenigstens ein Erfassungsmittel vorgesehen ist, das mit der Steuerungs- und/oder Regelungseinheit mittelbar und/oder unmittelbar verbunden ist und mittels dessen Werte zur Ermittlung der Dialysance oder Clearance wenigstens eines Stoffes mittelbar und/oder unmittelbar erfassbar sind. Des Weiteren betrifft die Erfindung ein Verfahren zum Betrieb einer Dialysemaschine mit den Merkmalen des Anspruchs 11. Danach ist vorgesehen, dass in einem ersten Schritt Parameter für die Dialysebehandlung eingegeben werden, wobei in einem zweiten Schritt unter Vorhaltung eines Modells zumindest unter Einbeziehung der zuvor eingegebenen Parameter für wenigstens eine Konzentration eines gelösten Stoffes im vaskulären Blutkreislauf des Patienten der durch die Dialysebehandlung zu erwartende Verlauf der Konzentrationsänderung und/oder der zu erwartende Endwert der Konzentration dieses Stoffes berechnet und ausgewertet wird, und wobei in einem dritten Schritt mittelbar und/oder unmittelbar in Abhängigkeit von der Auswertung des zu erwartenden Verlaufs der Konzentrationsänderung und/oder des zu erwartenden Endwertes der Konzentration des Stoffes über Ausgabemittel wenigstens ein Signal und/oder eine Anzeige ausgegeben wird und/oder die Dialysebehandlung bzw. die maschinenseitigen Einstellungen für die Dialysebehandlung mittelbar und/oder unmittelbar beeinflusst wird/werden.

Der Verfahrensschritt der Auswertung umfasst vorzugsweise die Erstellung einer Prognose des zu erwartenden Verlaufs der Konzentrationsänderung und/oder des zu erwartenden Endwertes der Konzentration des ausgewerteten Stoffes im vaskulären Blutkreislauf des Patienten während und in Folge der Dialysebehandlung.

Beispielsweise wird es durch den Einsatz von sog. Point-of-Care-Analysatoren immer mehr möglich, vor Behandlungsbeginn dem Patienten durch das Behandlungspersonal minimale Blutmengen zu entnehmen und mit diesen minimalen Blutmengen in kürzester Zeit die Konzentration verschiedener Stoffe im Patientenblut zu bestimmen. Da die Konzentration dieser Stoffe im Dialysat aus der Herstellerangabe und der Dosierung im Dialysegerät exakt bekannt ist, kann bei Kenntnis der Effizienz des Stoffaustauschs und den stoffspezifischen physiologischen Verhältnissen im Patienten die Kinetik der Stoffkonzentration im Patientenplasma mittels eines Modells abgeschätzt werden. Beim Erreichen oder bei der Prognose von kritischen Werten oder Änderungsraten für die Konzentration eines Stoffes oder einer Summengröße mehrerer Stoffe können vom Dialysegerät automatisch Maßnahmen eingeleitet werden. Diese können z. B. aus einer Warnung an den Benutzer bestehen, dass unter Verwendung des aktuellen Dialysats bei den gegebenen Behandlungsbedingungen akut oder im weiteren Verlauf der Dialyse mit Symptomen im Patienten zu rechnen ist. Ebenso kann automatisch die Dialyseeffizienz soweit vermindert werden, dass die mittels Modell berechnete Stoffkonzentration bzw. deren Änderungsrate unterhalb des für den Patienten kritischen Werts bleibt. Hierzu kann es auch nötig sein, den Stoffaustausch zwischen Patient und Dialysat zu unterbinden und nur mit einer Ultrafiltration (ISO-UF) fortzufahren. Alternativ kann dem Benutzer auch die Verwendung eines anderen Dialysats vorgeschlagen werden.

Es ist weiter denkbar, dass anhand eines eingespeicherten Grenzwertes für einen Parameter, bei dessen Über- oder Unterschreitung ein kritischer Wert vorliegt, insbesondere eines Grenzwerts für den zu erwartenden Verlauf und/oder den zu erwartenden Endwert der Konzentration des mindestens einen gelösten Stoffes im vaskulären Blutkreislauf des Patienten während oder in Folge der Dialysebehandlung, eine Über- oder Unterschreitung dieses Grenzwertes durch die Dialysemaschine vor und/oder im Verlauf der Dialysebehandlung erkannt wird.

Die kritischen Werte können z. B. anerkannten Richtlinien für Normwerte im Patienten oder Publikationen über die Toleranz von Konzentrationsverschiebungen im Patienten bei der Dialyse oder bei intensivmedizinischen Behandlungen entnommen werden und/oder patientenindividuelle Erfahrungswerte darstellen.

Ferner ist möglich, dass die Einstellung der Dialysemaschine für die Durchführung der Dialysebehandlung hinsichtlich Behandlungsdauer, Dialysatzusammensetzung, Einstellung der Flussraten im Blutkreislauf und/oder Dialysatkreislauf und/oder Einstellung der Art der Dialysebehandlung beeinflusst wird.

Darüber hinaus kann vorgesehen sein, dass nach Erkennung eines kritischen Wertes Informationen zu einsetzbaren Komponenten für die Dialysemaschine, wählbaren Parameterbereichen für die Dialysebehandlung und/oder Warnsignale ausgegeben werden und/oder ein Starten der Dialysebehandlung verhindert wird. Außerdem ist denkbar, dass bei Erkennung eines kritischen Wertes während der Dialysebehandlung eine Warnmeldung betreffend die gesonderte Bestimmung aktueller Blutparameter des Patienten ausgegeben wird, insbesondere hinsichtlich einer gesondert vorzunehmenden Bestimmung von Blutzucker, Calcium, Kalium, Natrium, Harnstoff, Alkohol und/oder pH-Wert und/oder von einem Dialyseverfahren auf ein Filtrationsverfahren, vorzugsweise Iso-Ultrafiltration, umgeschaltet wird. Besonders vorteilhaft ist es bei der Durchführung des Verfahrens zum Betrieb einer Dialysemaschine, wenn es sich bei der Dialysemaschine um eine Dialysemaschine gemäß einem der Ansprüche 1 bis 10 handelt.

Weitere Einzelheiten und Vorteile der vorliegenden Erfindung sollen nun anhand eines in der Zeichnung dargestellten Ausführungsbeispiels näher erläutert werden. Es zeigen:

Figur 1: eine schematische Darstellung einer Dialysemaschine;

Figur 2: ein Diagramm betreffend die Korrelation zwischen berechneter und gemessener Änderung der Plasma-Glucosekonzentration;

Figur 3: ein Diagramm betreffend die Korrelation zwischen berechneter und gemessener Änderung der Plasma-Kaliumkonzentration und

Figur 4: ein Diagramm betreffend die Korrelation zwischen berechneter und gemessener Änderung der Plasma-Bicarbonatkonzentration.

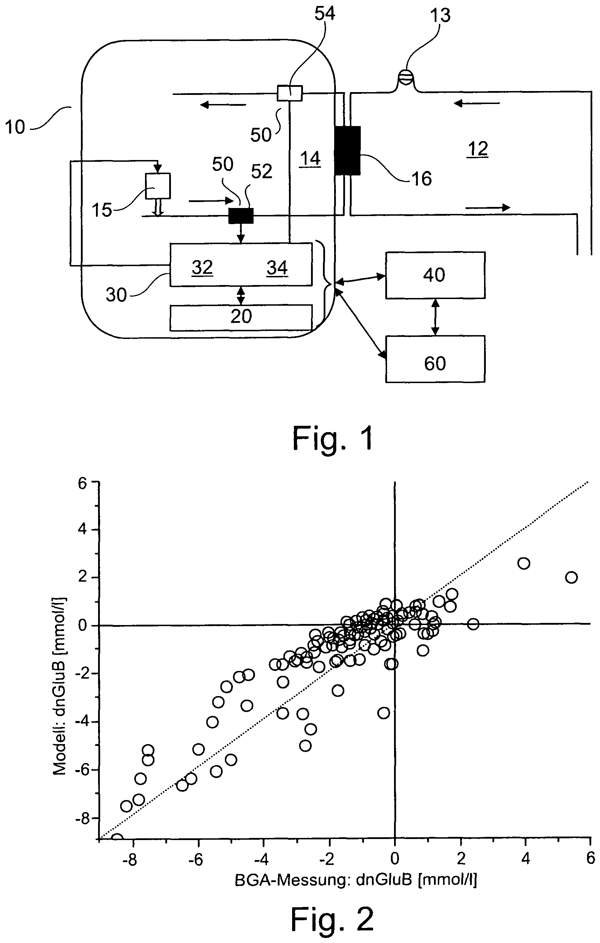

Figur 1 zeigt ein Ausführungsbeispiel einer erfindungsgemäßen Dialysemaschine 10. Grundsätzlich kann vorgesehen sein, dass die Vorrichtung zur Verwendung für eine Dialysemaschine 10 z. B. als Modul einer Dialysemaschine 10 ausgeführt ist und im wesentlichen die nachstehend beschriebenen Merkmale aufweist.

Am Benutzerinterface 20 bzw. den Eingabemitteln 20 der Dialysemaschine 10 können die für das Modell benötigten Parameter wie die initialen Blutwerte, das Verteilungsvolumen und die stoffspezifischen Warnschwellen eingegeben werden. Hierbei können dem Benutzer für jeden Stoff Vorgabewerte für die Art und Lage der Warnschwellen angeboten werden. Alternativ können patientenindividuelle Werte für die Warnschwellen von einem externen Speichermedium 40, z. B. einer Patientenkarte, gelesen werden. Auch sich nur langfristig ändernde Werte wie das Verteilungsvolumen können von dort gelesen werden. Des Weiteren erfolgt am Benutzerinterface 20 die Einstellung der Dialyseparameter. Auch werden hier ggf. die Warnungen beim Überschreiten einer Warnschwelle ausgegeben.

In einer vorteilhaften Ausführung können die Blutwerte vom Blutanalysesystem 60 entweder direkt über eine Datenverbindung oder indirekt über ein Speichermedium 40 an das Dialysegerät 10 übertragen werden.

Grundsätzlich kann vorgesehen sein, dass die Dialysemaschine 10 einen internen Speicher aufweist, der mit einem externen Speichermittel 40 verbindbar ist, so dass ein Datenaustausch stattfinden kann.

Die Dialysemaschine 10 weist weiter eine Maschinensteuerung 30 bzw. eine Auswerte- und Kontrolleinheit 30 auf, die eine Berechnungseinheit 32 und eine Steuerungs- und/oder Regelungseinheit 34 umfasst. Über die Maschinensteuerung 30 können die Kreislaufpumpen, insbesondere die dargestellte Pumpe 13 des Blutkreislaufs 12 sowie die nicht näher dargestellte Pumpe des Dialysatkreislaufs 14, aber auch Fültratpumpen angesteuert sowie deren aktuelle Betriebsparameter erfasst werden. Blutkreislauf 12 und Dialysatkreislauf 14 sind semipermeabel mittels eines Dialysators 16 verbunden.

Des Weiteren kann über die Maschinensteuerung 30 ein Dosiereinheit 15 angesteuert werden, mittels derer online die Zusammensetzung des Dialysats eingestellt werden kann. Die als Leitfähigkeitssensoren 52, 54 ausgeführten Erfassungsmittel 50 stehen ebenfalls mit der Maschinensteuerung 30 in Signalverbindung.

Eine wichtige Aufgabe der Maschinensteuerung 30 bzw. Auswerte- und Kontrolleinheit 30 ist die Ermittlung der stoffspezifischen Dialysance. Dazu werden von ihr in regelmäßigen Abständen Messungen der NaCl-Dialysance bzw. Urea-Clearance durchgeführt. Diese Messungen können beispielsweise über Leitfähigkeitsverfahren (OCM), oder durch optische oder enzymatische Konzentrationsmessungen im Dialysat erfolgen. Für die interessierenden Stoffe ist in der Berechnungseinheit 32 ein Umrechnungsalgorithmus hinterlegt, der aus der Urea-Clearance die stoffspezifische Dialysance berechnet.

Dem in der Berechnungseinheit 32 vorgehaltenen Modell liegen folgende Überlegungen zu Grunde und sind dementsprechend im Modell abgebildet:

Der Verlauf der Stoffkonzentration $c_{bi}$ im Blutplasma eines Patienten mit stoffspezifischem Verteilungsvolumen $V$ während der Dialyse gegen eine dialysatseitige Stoffkonzentration $c_{di}$ bei einer stoffspezifischen Dialysance $D$ und dem stoffspezifischen und von der Proteinkonzentration im Patienten abhängenden Donnanfaktor $\alpha$ kann bei einer Metabolisierungs- bzw. Generationsrate $g(t)$ genähert mittels eines 1-Kompartimente-Modells abgeschätzt werden (vgl. Gotch in Replacement of Renal Function by Dialysis):

$$\frac{dc_{bi}(t)}{dt} = -\frac{D}{V}\left(c_{bi}(t) - \frac{c_{di}}{\alpha}\right) + g(t)$$

Eine Lösung ist analytisch oder iterativ möglich und ist bei Vernachlässigung von $g(t)$ gegeben durch:

$$c_{bi}(t) = \frac{c_{di}}{\alpha} + \left(c_{bi}(0) - \frac{c_{di}}{\alpha}\right)\exp\left(-\frac{D}{V}t\right)$$

$$\Delta c_{bi} = c_{bi}(t) - c_{bi}(0) = \left(\frac{c_{di}}{\alpha} - c_{bi}(0)\right)\left(1 - \exp(-\frac{D}{V}t)\right)$$

Zur Berechnung von $c_{bi}(t)$ ist die Kenntnis der Werte der übrigen Parameter nötig:

- Die dialysatseitige Stoffkonzentration $c_{di}$ kann direkt aus den Herstellerangaben des zur Herstellung verwendeten Konzentrats und dem Mischungsverhältnis mit RO-Wasser berechnet werden. Dieses ist bei Verwendung von Dialysat aus einem Batch bekannt oder kann bei einer Online-Erzeugung laufend berechnet werden.

- Die stoffspezifische Dialysance D entspricht für die wichtigsten ein- und zweiwertigen Ionen (z. B. Calcium, Kalium) im Wesentlichen derjenigen von NaCl, dessen Dialysance durch eine Online-Clearance-Messung während der Dialyse laufend bestimmt werden kann. Für andere kleinere Moleküle (z. B. Bicarbonat, Glucose) kann deren Dialysance aus der NaCl-Dialysance und den Dialysebedingungen (insbesondere Blut- und Dialysatfluss) berechnet werden mit f<1.0. Die Werte für f sind aus der Literatur verfügbar oder können aus Labormessungen bestimmt werden.

- Der Donnanfaktor $\alpha$ kann für einwertige Kationen als konstant bei 0.95 angenommen werden.

- Das Verteilungsvolumen $V$ für Harnstoff und Natriumchlorid kann mittels Bioimpedanz-Messungen, durch "Kinetic Modelling" durch Messung der Harnstoff-Reduktionsrate, oder durch anthropometrische Formeln (z. B. Watson-Formel) ermittelt werden. Für andere Substanzen sollte ein geeigneter Korrekturfaktor zu V verwendet werden, der aus der Literatur zu entnehmen ist. Da V sich nur wenig zwischen den Dialysebehandlungen ändert, ist eine Bestimmung in längeren Intervallen (z. B. monatlich) ausreichend.

- Die Anfangskonzentration $c_{bi}(0)$ muss vor jeder Behandlung neu bestimmt werden, da von ihr aus der weitere Konzentrationsverlauf berechnet wird. Hierzu können Blutgasanalysatoren, Point-of-Care-Analysatoren oder z. B. Blutglucose-Messgeräte dienen.

- Die größte Unsicherheit im Modell stellt die Generationsrate $g(t)$ dar. Sie beschreibt sowohl die Erzeugung des betrachteten Stoffes durch Umsetzung aus anderen Stoffen (z. B. Glucose aus Glycogen, Urea als Stoffwechselprodukt) als auch den Transport des Stoffes aus oder in andere Kompartimente (z. B. Transport von Glucose und Kalium in die Zellen, Freisetzung von Calcium aus den Knochen). Zum Teil versuchen dabei körpereigene Regelmechanismen, die Stoffkonzentration innerhalb bestimmter Grenzen konstant zu halten. Gerade bei hocheffizienten Dialysebehandlungen kann aber im Normalfall in guter Näherung davon ausgegangen werden, dass die körpereigenen Mechanismen zur Konzentrationsänderung langsamer ablaufen als die durch die Dialyse bewirkten Änderungen. Daher kann $g(t)$ zunächst bei der Modellbildung vernachlässigt werden. Eine Verbesserung der Modellanpassung ist durch die Verwendung von Daten aus der Literatur oder durch patientindiviuduelle Untersuchungen möglich. Zudem ist denkbar, den zeitlichen Verlauf von $g(t)$ anhand eines Modells und weiterer patientenspezifischer Parameter (z. B. Insulingaben, Nahrungsaufnahme) abzuschätzen.

Nachdem somit alle Parameter des Modells definiert sind, müssen zudem Warnschwellen $c_{bi, krit}, \Delta c_{bi, krit}$ bzw.

$$\left( \frac{dc_{bi}}{dt} \right)_{krit}$$ für das Auslösen einer Reaktion des Dialysegeräts, z. B. die Ausgabe einer Warnung, definiert werden.

Bedingungen für eine Reaktion können z. B. sein:

- $c_{bi}(t) > c_{bi, krit}$ oder $c_{bi}(t) < c_{bi, kri}$ bzw. $|\Delta c_{bi}(t)| > \Delta c_{bi, krit}$ für den aktuellen Zeitpunkt $t$ nach Start der Dialyse, oder für einen beliebigen in der Zukunft liegenden Zeitpunkt $t$ nach Start der Dialyse vor dem geplanten Ende der Dialyse.

- $\left| \frac{dc_{bi}}{dt} \right| > \left| \left( \frac{dc_{bi}}{dt} \right)_{krit} \right|$ für den aktuellen Zeitpunkt $t$ nach Start der Dialyse, oder für einen beliebigen in der Zukunft liegenden Zeitpunkt $t$ nach Start der Dialyse vor dem geplanten Ende der Dialyse.

Hiervon ausgehend werden von der Steuerungs- und/oder Regelungseinheit 34 ggf. automatisiert Aktionen beim Überschreiten einer Warnschwelle ausgeführt. Dabei kann es sich um eine Anpassung der Dialyseeffizienz durch Korrektur des Blut- oder Dialysatflusses, den Wechsel der Behandlungsart (z. B. von HDF auf HD bzw. Wechsel auf ISO-UF), oder die Änderung der Dialysatzusammensetzung, z. B. durch Anpassung der Natrium-, Kalium- oder Bicarbonatkonzentration handeln.

Warnungen und Maßnahmen sind bei der Prognose einer möglichen Hypoglycämie, einer möglichen Hypocalciämie, einer möglichen Hypokaliämie, einer möglichen Alkalose, eines möglichen Disäquilibriums bei z. B. Harnstoff und/oder Alkohol und/oder einer möglichen Hyponatriämie denkbar und bei einer bevorzugten Ausführungsform einer Dialysemaschine 10 verwirklicht.

So kann beispielsweise bei der maschinenseitigen Prognose einer möglichen Unterschreitung des Grenzwertes für den Blutzucker aufgrund der maschinenseitigen Betriebsparameter folgendermaßen vorgegangen werden:

Dabei ist zunächst vorauszuschicken, dass, wie in klinischen Studien gezeigt, es bei Verwendung von glucosefreiem Dialysat sowohl bei diabetischen als auch bei nichtdiabetischen Patienten zu hypoglycämischen Episoden (Serum-Glucose < 70 mg/dl) kommen kann (Ref: Jackson et al., Clin. Nephrology 54, ". 30-40; Jackson et al., Clin. Nephrology 51, S. 242-247). Zugleich konnte gezeigt werden, dass die hormonelle Antwort auf den hypoglycämischen Zustand, die zu einer verstärkten Freisetzung von Glucose aus in der Leber gespeichertem Glycogen führen soll, während der Dialyse gehemmt ist, zum Teil durch die Entfernung von Glukagon über die Dialysemembran. Damit besteht besonders für multimorbide Patienten mit schlechtem Ernährungszustand die Gefahr hypoglycämischer Symptome während oder nach der Dialyse.

Der für die Anwendung des Modells essentielle Startwert der Plasma-Glucosekonzentration ist gerade bei Diabetikern aus den mehrmals täglich durchzuführenden Blutglucosemessungen leicht verfügbar, wobei eine Messung unmittelbar vor der Dialyse erfolgen sollte. Der Glucosegehalt des Dialysats ist aus den Herstellerangaben bekannt und kann bei Verwendung von Barcode oder RFID-Technologie automatisiert an das Dialysegerät übertragen werden. Die aktuelle Glucose-Dialysance kann aus der Online-Clearance-Messung während der Dialyse bestimmt werden und entspricht näherungsweise der Harnstoff-Clearance. Als Warnschwelle sollte der zur Definition der Hypoglycämie übliche Wert von 70 mg/dl verwendet werden.

Unter Verwendung der beschriebenen Parameter konnte in einer internen klinischen Studie gezeigt werden, dass die erwartete Änderung der Glucosekonzentration während einer Dialyse von ca. 4 h mit der gemessenen gut übereinstimmt, wobei allerdings alle Glucosekonzentrationen im Blutplasma oberhalb der Hypoglycämie-Schwelle lagen. Figur 2 zeigt die ermittelte Korrelation zwischen berechneter und gemessener Änderung der Plasma-Glucosekonzentration während der Hämodialysebehandlung, hier den Unterschied zwischen Wert zu Beginn und zu Ende der Behandlung. Die Abzissenwerte sind dabei die mittels Blutgasanalysator 40 ermittelten tatsächlichen Messwerte, während die Ordinatenwerte die prognostizierten Werte sind.

Eine Hypoglycämie-Warnung könnte dann z. B. über das Benutzerinterface 20 ausgegeben werden, wenn während der programmierten Dialysedauer bei Anwendung des Modells mit einem Absinken der Glucosekonzentration unter die Hypoglycämie-Schwelle zu rechnen ist. Dieses kann bereits vor Start der Dialyse geschehen. In diesem Fall könnte dem Anwender z. B. die Verwendung eines anderen Konzentrats vorgeschlagen werden.

Ebenfalls könnte zu dem Zeitpunkt, zu dem während der Dialyse die Hypoglycämie-Schwelle unterschritten wird, eine Warnung ausgegeben werden und der Anwender bzw. der Patient zu einer Blutzuckermessung am Patienten aufgefordert werden. Weiterhin könnte automatisiert die Dialyse beendet und mit einer isolierten Ultrafiltration fortgefahren werden.

Bei der Hypocalciämie-Warnung bzw. -Antizipation wird folgendermaßen vorgegangen:

Es wird analog wie bei der Hypoglycämie-Warnung bzw. -Antizipation vorgegangen, wobei eine Messung des Plasma-Calciums mit einem Blutanalysator 60 vor der Behandlung erfolgt. Der Wert des Dialysat-Calciums kann aus Herstellerangaben (z. B. Angaben auf dem Kanister) festgestellt und über das Benutzerinterface 20 oder über eine Datenkarte 40 eingegeben werden. Um die Warnung oder eine Beeinflussung bzw. Anpassung der maschinenseitigen Einstellungen für die Dialysebehandlung auszulösen, kann eine Hypocalciämie-Schwelle unter Verwendung des unteren Normwerts aus Literatur vorgesehen sein. Dadurch ergibt sich der Vorteil, dass einer bei Hypocalciämie auftretenden Störung der Erregbarkeit der Nerven und den hieraus resultierenden Krämpfe (Tetanie) sowie schlimmstenfalls Herzrythmusstörungen weit vor Erreichen eines kritischen Wertes vorgebeugt werden kann.

Die Hypokaliämie-Warnung bzw. -Antizipation kann folgendermaßen ablaufen:

Es wird analog wie bei der Hypoglycämie- und/oder Hypocalciämie-Warnung bzw. - Antizipation vorgegangen, wobei eine Messung des Plasma-Kaliums mit einem Blutanalysator 60 vor der Behandlung erfolgt. Der Wert des Dialysat-Kaliums wird aus Herstellerangaben (z. B. Angaben auf dem Kanister) festgestellt und über das Benutzerinterface 20 oder über eine Datenkarte 40 eingegeben.

Um die Warnung oder eine Beeinflussung bzw. Anpassung der maschinenseitigen Einstellungen für die Dialysebehandlung auszulösen, kann eine Hypokaliämie-Schwelle unter Verwendung des unteren Normwerts aus Literatur vorgesehen sein. Weiter kann neben einer absoluten Hypokaliämie-Schwelle eine Schwelle für den Betrag der Absenkung vom Startwert und/oder eine Schwelle für die Änderungsrate vorgesehen sein.

Durch ergibt sich der Vorteil, dass bei Hypokaliämie oder schneller Änderung der Plasma-Kaliumkonzentration einer möglicherweise auftretenden Herzrhythmusstörung, insbesondere bei Hypokaliämie einer Herzrhythmusstörung bis hin zu einem möglichen Kammerflimmern, rechtzeitig im Vorfeld vorgebeugt werden kann. Besteht im Dialysegerät 10 die Möglichkeit zur individuellen Dosierung einer kaliumhaltigen Komponente des Dialysats, so ist statt der Ausgabe einer Warnung auch die Regelung des Kaliumgehaltes des Dialysats unter Verwendung des Patientenmodells dergestalt möglich, dass das Erreichen einer Warnschwelle vermieden wird. Die individuelle Dosierung kann beispielsweise durch die Dosiereinheit 15 erfolgen.

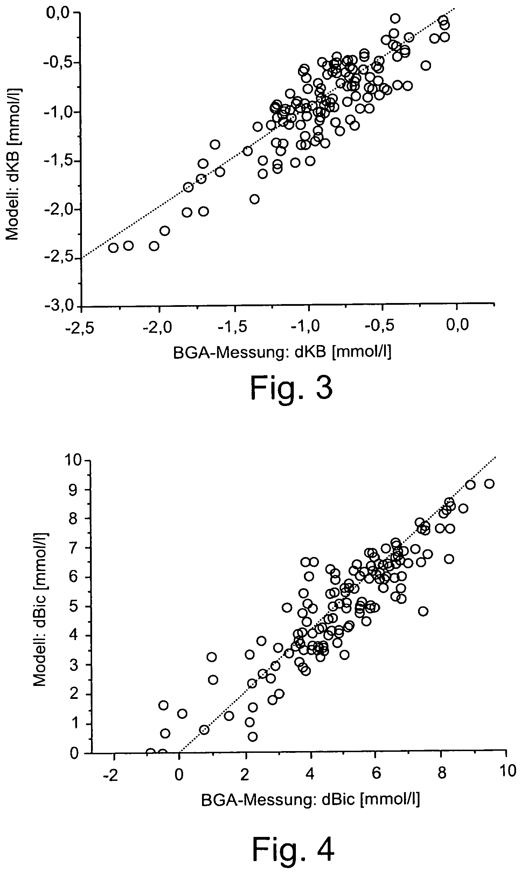

Unter Verwendung der beschriebenen Parameter konnte in einer internen klinischen Studie gezeigt werden, dass die erwartete Änderung der Kaliumkonzentration in Patientenplasma während einer Dialyse von ca. 4 h mit der gemessenen gut übereinstimmt. Figur 3 zeigt die ermittelte Korrelation zwischen berechneter und gemessener Änderung der Plasma-Kaliumkonzentration während der Hämodialysebehandlung, hier den Unterschied zwischen Wert zu Beginn und zu Ende der Behandlung. Die Abzissenwerte sind dabei die mittels Blutanalysator 60 ermittelten tatsächlichen Messwerte, während die Ordinatenwerte die prognostizierten Werte sind.

Die Alkalose-Warnung bzw. Antizipation betreffend Bicarbonat wird wie nachstehend beschrieben durchgeführt:

Auch hier wird analog den vorstehend beschriebenen Warn- und/oder Antizipationsmaßnahmen vorgegangen: Zunächst erfolgt eine Messung des Plasma-Bicarbonats mit einem Blutanalysator 60 vor der Behandlung. Der Wert des Dialysat-Bicarbonats kann aus Herstellerangaben, z. B. von den auf dem Kanister angegebenen Informationen oder der Bicarbonat-Sollkonzentration bei der Online-Herstellung des Dialysats aus Konzentrat entnommen und entsprechend eingegeben werden.

Ein Hauptziel der Dialyse ist die Korrektur der Azidose des Patienten durch Zufuhr von Bicarbonat während der Dialyse. Bei Patienten mit relativ hohem Plasma-Bicarbonat zu Beginn der Behandlung, z. B. bei Nierenrestfunktion, kann es bei langer hocheffizienter Dialyse mit hohem Bicarbonatgehalt im Dialysat zu einer Alkalose im Patienten kommen. Als Folge davon können eine stark verminderte Atmung, Kopfschmerzen sowie aufgrund der verstärkten Proteinbindung von Calcium bei gestiegenem pH-Wert Tetanie auftreten. Um dies zu verhindern, kann eine Alkalose-Schwelle, z. B. der obere Normwert aus der Literatur zuzüglich Offset für "vorweggenommene Azidose-Korrektur" bei Dialysepatienten verwendet werden.

Da bei Online-Aufbereitung des Dialysats im Dialysegerät 10 die Möglichkeit zur individuellen Dosierung einer bicarbonathaltigen Komponente besteht, so ist statt der Ausgabe einer Warnung auch die Regelung des Bicarbonatgehalts des Dialysats unter Verwendung des Patientenmodells dergestalt möglich, dass eine Alkalose vermieden wird.

Unter Verwendung der beschriebenen Parameter konnte in einer internen klinischen Studie gezeigt werden, dass die erwartete Änderung der Bicarbonatkonzentration in Patientenplasma während einer Dialyse von ca. 4 h mit der gemessenen gut übereinstimmt. Figur 4 zeigt die ermittelte Korrelation zwischen berechneter und gemessener Änderung der Plasma-Bicarbonatkonzentration während der Hämodialysebehandlung, hier den Unterschied zwischen Wert zu Beginn und zu Ende der Behandlung. Die Abzissenwerte sind dabei die mittels Blutanalysator 60 ermittelten tatsächlichen Messwerte, während die Ordinatenwerte die prognostizierten Werte sind.

Die Disäquilibrium-Warnung bzw. -Antizipation bei Urea, Alkohol kann, wie nachfolgend beschrieben, erfolgen:

Beide Stoffe, Harnstoff und Alkohol tragen zur Osmolalität des Plasmas bei und sind im Dialysat nicht vorhanden. Eine zu schnelle Verringerung der Konzentration im Blutplasma führt wegen des verzögerten Konzentrationsausgleichs im Gehirn zu einem Wassereinstrom ins Gehirn, was zu Hirnödemen führen kann. Hohe Ureakonzentrationen im Plasma können vor allem bei der Akutdialyse, bei neu an die Dialyse kommenden Patienten oder zu großen Behandlungsintervallen, etwa wenn eine Dialyse ausgelassen wurde, vorkommen. Die Messung der Urea-Konzentration ist beispielsweise mit einigen Point-of-Care-Analysatoren möglich.

Hohe Alkoholkonzentrationen im Plasma werden durch Alkoholmißbrauch hervorgerufen und können sowohl bei Dialysepatienten oder auch bei wegen Alkoholintoxikation auf die Intensivstation eingelieferten Patienten vorkommen. Die Bestimmung des Alkoholgehalts kann durch eine Blutprobe oder aus der Atemluft erfolgen.

Diese so ermittelten Werte für Harnstoff und/oder Alkohol können beispielsweise über das Benutzerinterface 20 eingegeben werden. Der Wert für das Dialysat ist bekanntermaßen Null und entsprechend voreingestellt. Als Warnschwellen können Schwellen für den Betrag der Absenkung vom Startwert und Schwellen für die zulässige Änderungsrate vorgegeben werden. Gegebenenfalls wird beispielsweise bei der Vorhersage oder dem Erreichen eines kritischen Wertes eine Warnung oder ein automatisches Umschalten auf Iso-Ultrafiltration eingeleitet.

Weiter kann eine Warnung bzw. Antizipation bei Hyponatriämie-Korrektur ermöglicht werden:

Aus der Intensivmedizin ist bekannt, dass eine zu schnelle Korrektur einer Hyponatriämie zu Störungen des Zentralen Nervensystems führen kann (z. B. zentrale pontine Myelinolyse).

Daher wird hier bei der Behandlung, die meist in einer Infusion einer NaCl-Lösung besteht, darauf geachtet, dass die Änderungsrate des Plasma-Natriums nicht über 0,5 mmol/l pro Stunde liegt. Statt einer NaCl-Infusion ist eine Hyponatriämie-Korrektur natürlich auch mittels Hämodialyse möglich. In einer internen klinischen Studie konnte gezeigt werden, dass sich die Änderung des Plasma-Natriums bei Verwendung des oben beschriebenen Modells gut vorhersagen lässt. Der Natriumgehalt des Dialysats kann vom Anwender in weiten Grenzen vorgegeben werden. Ein Initialwert des Plasma-Natriums kann z. B. aus der Labormessung stammen, mittels derer die Hyponatriämie diagnostiziert wurde. Wird unter Verwendung des oben beschriebenen Modells festgestellt, dass die Änderungsrate des Plasma-Natriums die kritische Schwelle überschreitet bzw. überschreiten wird, so kann der Benutzer zur Änderung des Dialysatnatriums aufgefordert werden.

Alternativ kann das Dialysegerät 10 automatisch die Na-Konzentration des Dialysats so anpassen, dass eine Überschreitung der kritischen Schwelle vermieden wird, oder nur Eingaben von Na-Konzentrationen durch den Benutzer zulassen, die nicht zu einer Überschreitung der kritischen Schwelle führen.

**Patentansprüche**

1. Dialysemaschine (10) mit

   - wenigstens einem Eingabemittel (20), das ausgebildet ist, um die Eingabe von Parametern für wenigstens eine Konzentration wenigstens eines gelösten Stoffes im vaskulären Blutkreislauf des Patienten zu ermöglichen;
   - einer Berechnungseinheit (32), die ausgebildet ist, um unter Vorhaltung eines Modells zumindest unter Einbeziehung der zuvor eingegebenen Parameter oder einer auf den Parametern basierenden iterativen Berechnung für wenigstens eine Konzentration dieses gelösten Stoffes im vaskulären Blutkreislauf des Patienten in Form der Anfangskonzentration des gelösten Stoffes und des Verteilungsvolumens des Patienten sowie der Dialysance oder der Clearance dieses Stoffes den durch die Dialysebehandlung zu erwartenden Verlauf der Konzentrationsänderung und/oder zu erwartenden Endwert der Konzentration dieses Stoffes im vaskulären Blutkreislauf des Patienten zu berechnen und auszuwerten; und
   - einer Steuer- und/oder Regelungseinheit (34), die ausgebildet ist, um in Abhängigkeit von dieser Auswertung über Ausgabemittel (20) wenigstens ein Signal und/oder eine Anzeige auszugeben und/oder die Dialysebehandlung zu beeinflussen; wobei
   - die Steuerungs- und/oder Regelungseinheit (34) dazu ausgebildet ist, die maschinenseitigen Einstellungen für die Dialysebehandlung, hinsichtlich Behandlungsdauer, Dialysatzusammensetzung, Einstellung der Flussraten im Blutkreislauf und/oder Dialysatkreislauf und/oder Einstellung der Art der Dialysebehandlung zu beeinflussen, wobei vorzugsweise die Dialysatzusammensetzung durch Ansteuerung einer Dosiereinheit (15) für Konzentrat von Dialysatbestandteilen und/oder die Flussraten durch Ansteuerung von Pumpen (13) des Blutkreislaufs und/oder Dialysatkreislaufs beeinflusst wird.

2. Dialysemaschine (10) nach Anspruch 1, **dadurch gekennzeichnet, dass** eine Speichereinheit (40) vorgesehen ist, in der wenigstens ein Grenzwert für einen Parameter hinterlegt ist, bei dessen Über- oder Unterschreitung ein kritischer Wert vorliegt, insbesondere Grenzwerte für zu erwartenden Verlauf und/oder zu erwartenden Endwert der Konzentration des wenigstens einen gelösten Stoffes, wobei zwischen Speichereinheit (40) und Berechnungseinheit (32) eine Datenaustauschverbindung besteht und wobei die Berechnungseinheit (32) ausgebildet ist, um zu erkennen, ob eine Unter- oder Überschreitung des Grenzwerts vorliegt und/oder zu erwarten ist.

3. Dialysemaschine (10) nach Anspruch 1 oder 2, **dadurch gekennzeichnet, dass** das Eingabemittel (20) ausgebildet ist, um die Eingabe initialer Blutwerte und/oder Grenzwerte für einen Parameter zu ermöglichen.

4. Dialysemaschine (10) nach Anspruch 2 oder 3, **dadurch gekennzeichnet, dass** die Speichereinheit (40) ein entnehmbares Speichermedium umfasst und/oder mit einem entnehmbaren Speichermedium verbindbar ist, wobei auf dem entnehmbaren Speichermedium wenigstens ein Parameter für die Dialysebehandlung hinterlegt ist.

5. Dialysemaschine (10) nach einem der vorhergehenden Ansprüche, **dadurch gekennzeichnet, dass** die Ausgabemittel (20) ausgebildet sind, um Informationen zu einsetzbaren Einmalkomponenten für die Dialysemaschine (10), wählbaren Parameterbereichen für die Dialysebehandlung und/oder Warnsignale auszugeben.

6. Dialysemaschine (10) nach einem der vorhergehenden Ansprüche, **dadurch gekennzeichnet, dass** die Steuerungs- und/oder Regelungseinheit (34) ausgebildet ist, um die Dialysebehandlung abzuschalten und/oder ein Starten der Dialysebehandlung zu verhindern.

7. Dialysemaschine (10) nach einem der vorhergehenden Ansprüche 2 bis 6, **dadurch gekennzeichnet, dass** der wenigstens eine kritische Wert ein Wert ist, bei dem Hypoglycämie, Hypocalciämie, Hypokaliämie, Alkalose bedingt durch Bikarbonat, ein Disäquilibrium, insbesondere ein Disäquilibrium hinsichtlich Harnstoff und/oder Blutalkohol, und/oder Hyponatriämie beim zu behandelnden Patienten eintritt.

8. Dialysemaschine (10) nach Anspruch 7, **dadurch gekennzeichnet, dass** die Steuer- und/oder Regelungseinheit (34) ausgebildet ist, um bei Erkennung eines kritischen Wertes eine Warnmeldung und/oder eine Vorschlagsmeldung betreffend einen Konzentratwechsel für das Dialysat auszugeben und/oder den Start der Dialysebehandlung zu blockieren.

9. Dialysemaschine (10) nach einem der vorhergehenden Ansprüche, **dadurch gekennzeichnet, dass** die Steuer- und/oder Regelungseinheit (34) ausgebildet ist, um die Dialysemaschine (10) bei Erkennung eines kritischen Wertes von einem Dialyseverfahren auf ein Filtrationsverfahren, vorzugsweise Iso-Ultrafiltration, umzuschalten.

10. Dialysemaschine (10) nach einem der vorhergehenden Ansprüche, **dadurch gekennzeichnet, dass** wenigstens ein Erfassungsmittel vorgesehen ist, das mit der Steuerungs- und/oder Regelungseinheit (34) verbunden ist und das ausgebildet ist, um die Dialysance oder Clearance wenigstens eines Stoffes zu erfassen.

11. Verfahren zum Betrieb einer Dialysemaschine (10) wobei

- in einem ersten Schritt Parameter für die Dialysebehandlung über ein Eingabemittel der Dialysemaschine eingegeben werden,
- in einem zweiten Schritt, in einer Berechnungseinheit der Dialysemaschine, unter Vorhaltung eines Modells zumindest unter Einbeziehung der zuvor eingegebenen Parameter oder einer auf den Paramtern basierenden iterativen Berechnung für wenigstens eine Konzentration wenigstens eines gelösten Stoffes im vaskulären Blutkreislauf des Patienten in Form der Anfangskonzentration des gelösten Stoffes und des Verteilungsvolumens des Patienten sowie der Dialysance oder der Clearance dieses Stoffes der durch die Dialysebehandlung zu erwartende Verlauf der Konzentrationsänderung und/oder der zu erwartende Endwert der Konzentration dieses Stoffes im vaskulären Blutkreislauf des Patienten berechnet und ausgewertet wird, und
- in einem dritten Schritt in Abhängigkeit dieser Auswertung ein Signal und/oder eine Anzeige ausgegeben wird.

12. Verfahren nach Anspruch 11, **dadurch gekennzeichnet, dass** anhand eines eingespeicherten Grenzwertes für einen Parameter, bei dessen Über- oder Unterschreitung ein kritischer Wert vorliegt, insbesondere Grenzwerte für zu erwartenden Verlauf und/oder zu erwartenden Endwert der Konzentration des gelösten Stoffes, durch die Dialysemaschine (10) erkannt wird, ob eine Unter- oder Überschreitung des Grenzwerts vorliegt und/oder zu erwarten ist.

13. Verfahren nach Anspruch 12, **dadurch gekennzeichnet, dass** nach Erkennung eines kritischen Wertes

- Informationen zu einsetzbaren Einmalkomponenten für die Dialysemaschine (10), wählbaren Parameterbereichen für die Dialysebehandlung und/oder Warnsignale ausgegeben werden und/oder ein Starten der Dialysebehandlung verhindert wird; und/oder

- eine Warnmeldung betreffend die Bestimmung aktueller Blutparameter des Patienten ausgegeben wird, insbesondere hinsichtlich einer gesondert vorzunehmenden Bestimmung von Blutzucker, Calcium, Kalium, Natrium, Harnstoff, Alkohol und/oder pH-Wert.

**Claims**

1. A dialysis machine (10) comprising

- at least one input means (20) that is configured to enable the input of parameters for at least one concentration of at least one dissolved substance in the vascular blood circuit of the patient;

- a calculation unit (32) that is configured to calculate and evaluate the development of the concentration change to be expected by the dialysis treatment and/or the end value of the concentration of said dissolved substance to be expected in the vascular blood circuit of the patient while providing a model at least including the previously input parameters or including an iterative calculation based on said parameters for at least one concentration of said dissolved substance in the vascular blood circuit of the patient in the form of the starting concentration of said dissolved substance and of the distribution volume of the patient, and including the dialysance or the clearance of said substance; and

- control and/or regulation unit (34) that is configured to output at least one signal and/or one display via output means (20) and/or to influence the dialysis treatment in dependence on this evaluation; wherein

- the control and/or regulation unit (34) is configured to influence the settings for the dialysis treatment at the machine side with respect to the treatment duration, the dialyzate composition, the setting of the flow rates in the blood circuit and/or dialyzate circuit and/or the setting of the type of the dialysis treatment, wherein the dialyzate composition is preferably influenced by a control of a metering unit (15) for a concentrate of dialyzate components and/or wherein the flow rate is influenced by a control of pumps (13) of the blood circuit and/or dialyzate circuit.

2. A dialysis machine (10) in accordance with claim 1, **characterized in that** a storage unit (40) is provided in which at least one limit value for a parameter is stored on whose exceeding or falling below a critical value is present, in particular limit values for the development to be expected and/or for the end value of the concentration of the at least one dissolved substance to be expected, with there being a data exchange connection between the storage unit (40) and the calculation unit (32) and with the calculation unit (32) being configured to recognize whether a falling below or exceeding of the limit value is present and/or to be expected.

3. A dialysis machine (10) in accordance with either of claims 1 or 2, **characterized in that** the input means (20) is configured to enable the input of initial blood values and/or limit values for a parameter.

4. A dialysis machine (10) in accordance with either of claims 2 or 3, **characterized in that** the storage unit (40) includes a removable storage medium and/or can be connected to a removable storage medium, with at least one parameter for the dialysis treatment being stored on the removable storage medium.

5. A dialysis machine (10) in accordance with one of the preceding claims, **characterized in that** the output means (20) are configured to output information on usable disposable components for the dialysis machine (10), selectable parameter ranges for the dialysis treatment and/or warning signals.

6. A dialysis machine (10) in accordance with one of the preceding claims, **characterized in that** the control and/or regulation unit (34) is configured to switch off the dialysis treatment and/or prevent a starting of the dialysis treatment.

7. A dialysis machine (10) in accordance with one of the preceding claims 2 to 6, **characterized in that** the at least one critical value is a value at which hypoglycemia, hypocalcemia, hypokalemia, alkalosis caused by bicarbonate, a disequilibrium, in particular a disequilibrium with respect to urea and/or blood alcohol, and/or hyponatriemia occurs in the patient to be treated.

8. A dialysis machine (10) in accordance with claim 7 **characterized in that** the control and/or regulation unit (34) is configured to output a warning message and/or a proposal message relating to a concentrate change for the dialyzate and/or to block the start of the dialysis treatment when a critical value is recognized.

9. A dialysis machine (10) in accordance with one of the preceding claims, **characterized in that** the control and/or regulation unit (34) is configured to switch the dialysis machine (10) from a dialysis method to a filtration method, preferably to isolated ultrafiltration, when a critical value is recognized.

10. A dialysis machine (10) in accordance with one of the preceding claims, **characterized in that** at least one detection means is provided which is connected to the control and/or regulation unit (34) and is configured to detect the dialysance or clearance of at least one substance.

11. A method for the operation of a dialysis machine (10), wherein,

- in a first step, parameters for the dialysis treatment are input via an input means of the dialysis machine,
- in a second step, the development of the concentration change to be expected by the dialysis treatment and/or the end value of the concentration of said dissolved substance to be expected in the vascular blood circuit of the patient is/are calculated and evaluated in a calculation unit of the dialysis machine while providing a model at least including the previously input parameters or including an iterative calculation based on said parameters for at least one concentration of at least one dissolved substance in the vascular blood circuit of the patient in the form of the starting concentration of said dissolved substance and of the distribution volume of the patient, and including the dialysance or the clearance of said substance; and
- in a third step, a signal and/or display is/are output in dependence on this evaluation.

12. A method in accordance with claim 11, **characterized in that** it is recognized by the dialysis machine (10) with reference to a stored limit value for a parameter on whose exceeding or falling below a critical value is present, in particular limit values for the development to be expected and/or an end value to be expected of the concentration of the dissolved substance whether a falling below or an exceeding of the limit value is present and/or is to be expected.

13. A method in accordance with claim 12, **characterized in that**, after recognition of a critical value,

- information on usable disposable components for the dialysis machine (10), on selectable parameter ranges for the dialysis treatment and/or warning signals is output and/or a starting of the dialysis treatment is prevented; and/or
- a warning message relating to the determination of then current blood parameters of the patient is output, in particular with respect to a determination to be carried out separately of blood sugar, calcium, potassium, sodium, urea, alcohol and/or pH.

**Revendications**

1. Dialyseur (10) comprenant

- au moins un moyen de saisie (20), qui est conçu pour permettre la saisie de paramètres pour au moins une concentration d'au moins une substance dissoute dans le circuit sanguin vasculaire du patient ;
- une unité de calcul (32), qui est conçue pour calculer et évaluer, avec la mise à disposition d'un modèle compte tenu au moins des paramètres saisis auparavant ou d'un calcul itératif se basant sur les paramètres pour au moins une concentration de cette substance dissoute dans le circuit sanguin vasculaire du patient sous la forme de la concentration initiale de la substance dissoute et du volume de distribution du patient ainsi que de la dialysance ou de la clairance de cette substance, l'évolution à prévoir du changement de la concentration à travers le traitement de dialyse et/ou la valeur finale à prévoir de la concentration de cette substance dans le circuit sanguin vasculaire du patient ; et
- une unité de commande et/ou de régulation (34), qui est conçue pour, en fonction de cette évaluation, sortir au moins un signal et/ou un affichage par le biais de moyens de sortie (20) et/ou influencer le traitement de dialyse ; dans lequel
- l'unité de commande et/ou de régulation (34) est conçue pour influencer les réglages du dialyseur pour le traitement de dialyse, en ce qui concerne la durée de traitement, la composition du dialysat, le réglage des débits dans le circuit sanguin et/ou le circuit de dialysat et/ou le réglage du type de traitement de dialyse, dans lequel de préférence, la composition du dialysat est influencée par la commande d'une unité de dosage (15) pour concentré de constituants de dialysat et/ou les débits sont influencés par la commande de pompes (13) du circuit sanguin et/ou du circuit de dialysat.

2. Dialyseur (10) selon la revendication 1, **caractérisé en ce qu'**une unité de mémoire (40) est prévue, dans laquelle est enregistrée au moins une valeur limite pour un paramètre, dont un dépassement vers le haut ou vers le bas entraîne la présence d'une valeur critique, en particulier des valeurs limites pour l'évolution à prévoir et/ou la valeur finale à prévoir de la concentration de l'au moins une substance dissoute, une liaison d'échange de données existant entre l'unité de mémoire (40) et l'unité de calcul (32) et l'unité de calcul (32) étant conçue pour détecter s'il y a et/ou s'il faut prévoir un dépassement vers le haut ou vers le bas de la valeur limite.

3. Dialyseur (10) selon la revendication 1 ou 2, **caractérisé en ce que** le moyen de saisie (20) est conçu pour permettre la saisie de taux sanguins initiaux et/ou de valeurs limites pour un paramètre.

4. Dialyseur (10) selon la revendication 2 ou 3, **caractérisé en ce que** l'unité de mémoire (40) comprend un support de mémoire qui peut être retiré et/ou peut être raccordée à un support de mémoire qui peut être retiré, au moins un paramètre pour le traitement de dialyse étant enregistré sur le support de mémoire qui peut être retiré.

5. Dialyseur (10) selon l'une des revendications précédentes, **caractérisé en ce que** les moyens de sortie (20) sont conçus pour sortir des informations sur des composants à usage unique utilisables pour le dialyseur (10), sur des plages de paramètre sélectionnables pour le traitement de dialyse et/ou des signaux d'avertissement.

6. Dialyseur (10) selon l'une des revendications précédentes, **caractérisé en ce que** l'unité de commande et/ou de régulation (34) est conçue pour arrêter le traitement de dialyse et/ou empêcher un démarrage du traitement de dialyse.

7. Dialyseur (10) selon l'une des revendications précédentes 2 à 6, **caractérisé en ce que** l'au moins une valeur critique est une valeur pour laquelle une hypoglycémie, une hypocalcémie, une hypokaliémie, une alcalose due au bicarbonate, un déséquilibre, en particulier un déséquilibre concernant l'urée et/ou le taux d'alcool dans le sang, et/ou une hyponatrémie se produit chez le patient à traiter.

8. Dialyseur (10) selon la revendication 7, **caractérisé en ce que** l'unité de commande et/ou de régulation (34) est conçue pour, lors de la détection d'une valeur critique, sortir un message d'avertissement et/ou un message de suggestion relatif à un changement de concentré pour le dialysat et/ou bloquer le démarrage du traitement de dialyse.

9. Dialyseur (10) selon l'une des revendications précédentes, **caractérisé en ce que** l'unité de commande et/ou de régulation (34) est conçue pour, lors de la détection d'une valeur critique, commuter le dialyseur (10) d'un procédé de dialyse à un procédé de filtration, de préférence d'ultrafiltration isolée.

10. Dialyseur (10) selon l'une des revendications précédentes, **caractérisé en ce qu'**au moins un moyen de détection est prévu, qui est raccordé à l'unité de commande et/ou de régulation (34) et qui est conçu pour détecter la dialysance ou la clairance d'au moins une substance.

11. Procédé de fonctionnement d'un dialyseur (10), dans lequel

- dans une première étape, des paramètres pour le traitement de dialyse sont saisis par le biais d'un moyen de saisie du dialyseur,
- dans une seconde étape, dans une unité de calcul du dialyseur, avec la mise à disposition d'un modèle compte tenu au moins des paramètres saisis auparavant ou d'un calcul itératif se basant sur les paramètres pour au moins une concentration d'au moins une substance dissoute dans le circuit sanguin vasculaire du patient sous la forme de la concentration initiale de la substance dissoute et du volume de distribution du patient ainsi que de la dialysance ou de la clairance de cette substance, l'évolution à prévoir du changement de la concentration à travers le traitement de dialyse et/ou la valeur finale à prévoir de la concentration de cette substance dans le circuit sanguin vasculaire du patient est calculée et évaluée, et
- dans une troisième étape, un signal et/ou un affichage est sorti en fonction de cette évaluation.

12. Procédé selon la revendication 11, **caractérisé en ce que**, à l'aide d'une valeur limite enregistrée pour un paramètre, dont un dépassement vers le haut ou vers le bas entraîne la présence d'une valeur critique, en particulier des valeurs limites pour l'évolution à prévoir et/ou la valeur finale à prévoir de la concentration de la substance dissoute, il est détecté par le dialyseur (10) si un dépassement vers le haut ou vers le bas de la valeur limite est présent et/ou à prévoir.

**13.** Procédé selon la revendication 12, **caractérisé en ce que**, après la détection d'une valeur critique,

- des informations sur des composants à usage unique utilisables pour le dialyseur (10), sur des plages de paramètres sélectionnables pour le traitement de dialyse et/ou des signaux d'avertissement sont sortis et/ou un démarrage du traitement de dialyse est empêché ; et/ou
- un message d'avertissement relatif à la détermination de paramètres sanguins actuels du patient est sorti, en particulier concernant une détermination à effectuer séparément de la glycémie, du calcium, du potassium, du sodium, de l'urée de l'alcool et/ou de la valeur de pH.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20020099282 A1
- US 20030208113 A1
- US 5091094 A
- EP 0942759 B1
- EP 0942759 A

**In der Beschreibung aufgeführte Nicht-Patentliteratur**

- **JACKSON et al.** *Clin. Nephrology,* vol. 54, 30-40
- **JACKSON et al.** *Clin. Nephrology,* vol. 51, 242-247